# EUROPEAN PATENT SPECIFICATION

(11) **EP 2 178 571 B1**
(45) Date of publication and mention of the grant of the patent: **08.06.2011**
(21) Application number: 08829398.0
(22) Date of filing: 31.07.2008
(51) Int. Cl.: A61L 12/14

(54) **OPHTHALMIC COMPOSITIONS COMPRISING A CARBOXYL-MODIFIED FRUCTAN OR A SALT THEREOF**
OPHTHALMISCHE ZUSAMMENSETZUNGEN MIT EINEM CARBOXYLMODIFIZIERTEM FRUCTAN ODER EINEM SALZ DAVON
COMPOSITIONS OPHTALMIQUES COMPRENANT UN FRUCTANE MODIFIÉ PAR CARBOXYLE OU UN SEL DE CE DERNIER

(30) Priority: 02.08.2007 US 953535 P; 28.07.2008 US 180694
(43) Date of publication of application: 28.04.2010
(73) Proprietor: Bausch & Lomb Incorporated, Rochester, NY 14604-2701 (US)
(72) Inventor: MACLEOD, Steven, K., Henrietta, NY 14467 (US)
(74) Representative: Glas, Holger
(86) International application number: PCT/US2008/071675
(87) International publication number: WO 2009/032433

(56) References cited:
- WO-A-01/00771
- WO-A-96/06644
- WO-A-98/25972
- WO-A-99/64143
- WO-A-2006/038080
- US-A1- 2004 034 042

## Description

The invention relates to an ophthalmic composition comprising a carboxyl-modified fructan or a salt thereof, and the use of the composition to disinfect or package a contact lens, or as an eye drop product to comfort irritated eyes or rewet a contact lens.

### Background of the Invention

During normal use, contact lenses become soiled or contaminated with a wide variety of compounds that can degrade lens performance. For example, a contact lens will become soiled with biological materials such as proteins or lipids that are present in the tear fluid and which adhere to the lens surface. Also, by handling of the contact lens, sebum (skin oil) or cosmetics or other materials can soil the contact lens. These biological and external contaminants can affect visual acuity and patient comfort. Accordingly, it is important to remove any debris from the lens surface for continued comfortable use with a lens care solution that contains one or more cleaning components.

Ophthalmic compositions formulated as a lens care solution must also contain one or more disinfectant components. Presently, the two most popular disinfectant components are poly(hexamethylene biguanide), at times referred to as PHMB or PAPB, and polyquaternium-1.

PHMB-based care solutions represent a significant improvement in patient comfort and antimicrobial effectiveness compared to most other antimicrobial components. However, as with any antimicrobial component there remains a tradeoff between the concentration of the antimicrobial component in the solution and the comfort experienced by the patient. Due to its wide commercial acceptance, extensive efforts have been directed to improve the antimicrobial efficacy or the comfort level to the patient by chemically modifying PHMB.

An alternative approach to improving patient comfort has been the introduction of comfort agents or hydrating agents to lens care solutions. For example, U.S. Patent No. 7,135,442 describes the use of dexpanthenol in combination with the sugar alcohol, sorbitol. Other lens care products are known i.a. from WO 2006/038080, WO 96/06644 and US 2004/0034042.

From the documents WO 01/00771, WO 99/64143 and WO 98/25972 it is known to employ carboxyl-modified fructan in compositions to be used in other technical areas.

### Summary of the Invention

The invention is directed to an ophthalmic composition including a carboxyl-modified fructan or a salt thereof as defined in claim 7. An ophthalmic composition comprising a carboxyl-modified fructan or salt thereof can be used in an eye care product or a contact lens care product such as a contact lens packaging solution or a contact lens disinfecting solution as defined in claim 1.

### Detailed Description of the Invention

Applicants and others at Bausch & Lomb have developed and tested numerous ophthalmic compositions for use as lens care solutions. As mentioned above, such lens care solutions must satisfy a number of functional characteristics. First, the solutions must possess the cleaning ability to remove denatured tear proteins and tear lipids as well as other external contaminants. Second, the solutions must possess significant disinfecting ability against a number of different bacteria and fungal strains. Third, the solutions must remain comfortable to the contact lens patient with minimal stinging as well as provide a platform to provide additional comfort or protection to the ocular surface. Fourth, the solutions must not cause significant shrinkage or swelling of the many different contact lens materials, which in turn can lead to loss in visual acuity and unwanted or pronounced lens movement.

The invention is directed to an ophthalmic composition comprising a carboxyl-modified fructan or a salt thereof as defined in claim 7. As used herein, the term "fructan" is understood to include all oligosaccharides and polysaccharides that have a majority of anhydrofructose units. The fructan can have a polydisperse chain length distribution and can be straight-chain or branched. The fructans include primarily β-2,6 bonds as in levan, or β-2,1 bonds as in inulin. As used herein, the term "ophthalmic composition" denotes a composition intended for application in the eye or intended for treating a device to be placed in contact with the eye such as a contact lens.

In many embodiments, the carboxyl-modified fructan includes 0.3 to 3 carboxyl groups per anhydrofructose unit. In particular, the carboxyl-modified fructan includes at least 0.8 carboxyl groups per anhydrofructose unit, e.g., from I to 2.2 carboxyl groups per anhydrofructose unit. The carboxyl groups can be present in the form of carboxyalkyl groups, for example, but not limited to, carboxymethyl, carboxyethyl, dicarboxymethyl or carboxyethoxycarbonyl groups. The carboxyl-modified fructans can be obtained by etherification of the fructan using synthetic methods well known in the art. Moreover, the carboxyl groups can also be present in the form of oxidized hydroxymethylene or hydroxymethyl groups. Any one mixture of different carboxyl-modified fructans can also be used. Also, the carboxyl-modified fructan can be a mixed carboxyl derivative, which can be prepared by etherfication of the fructan to a carboxymethylated form. The carboxymethylated form is then oxidized. The reverse reaction sequence is also possible. Carboxymethylinulin (CMI) is one of the more preferred carboxyl-modified fructans.

Carboxymethylinulin (CMI) with a DS (degree of substitution) of 0.15-2.5 is disclosed in WO 95/15984 and in the article by Verraest DL, et al. "Carboxymethyl inulin: a New Inhibitor for Calcium Carbonate Precipitation," Journal of the American Oil Chemists' Society, 73 pp. 55-62 (1996). As described, CMI can be prepared by the reaction of a concentrated solution of inulin with sodium chloroacetate at an elevated temperature. Carboxylethylinulin (CEI) is disclosed in WO 96/34017. The oxidation of inulin is disclosed in WO 91/17189 and WO 95/12619 (C3-C4 oxidation, leading to dicarboxyinulin, DCI) and WO 95/07303 (C6 oxidation).

The carboxyl-modified fructan has an average chain length (degree of polymerisation, DP) of at least 3, that is from 3 to 1000 monosaccharide units. More likely, the average chain length is from 6 to 60 monosaccharide units.

In some instances, one can prepare the carboxyl-modified fructan by first modifying the fructan itself. For example, the fructan can have its chain length enzymatically extended prior to carboxylation. Alternatively, the fructan can have its chain length shortened through a hydrolysis reaction. Fructans of a select chain length range can then be isolated by fractionation. Fractionation of fructans such as inulin can be effected by, for example, low temperature crystallisation (see WO 94/01849), column chromatography (see WO 94/12541), membrane filtration (see EP-A 440 074 and EP-A 627 490) or selective precipitation with alcohol. Hydrolysis to produce shorter fructans can, for example, be effected enzymatically (endo-inulinase), chemically (water and acid) or by heterogeneous catalysis (acid column).

In addition to the carboxyl-modified fructan, the ophthalmic compositions can also include other biopolymers or derivatives of biopolymers. It is believed that one or more of these biopolymers protect the ocular surface, enhance patient comfort profiles and/or help maintain a hydrated lens surface. The concentration of the bioipolymers in the compositions is from 0.05 %w/v to 0.6 %w/v or from 0.05 %w/v to 0.3 %w/v.

One such biopolymer recognized in the ophthalmic community to alleviate ocular irritation is hyaluronic acid. Hyaluronic acid is a linear polysaccharide (long-chain biological polymer) formed by repeating disaccharide units consisting of D-glucuronic acid and N-acetyl-D-glucosamine linked by β(1-3) and β(1-4) glycosidic linkages. Hyaluronic acid is distinguished from the other glycosaminoglycans, as it is free from covalent links to protein and sulphonic groups. Hyaluronic acid is ubiquitous in animals, with the highest concentration found in soft connective tissue. It plays an important role for both mechanical and transport purposes in the body; e.g., it gives elasticity to the joints and rigidity to the vertebrate disks, and it is also an important component of the vitreous body of the eye.

Hyaluronic acid is accepted by the ophthalmic community as a compound that can protect biological tissues or cells from compressive forces. Accordingly, hyaluronic acid has been proposed as one component of a viscoelastic ophthalmic composition for cataract surgery. The viscoelastic properties of hyaluronic acid, that is, hard elastic under static conditions though less viscous under small shear forces enables hyaluronic acid to basically function as a shock absorber for cells and tissues. Hyaluronic acid also has a relatively large capacity to absorb and hold water. The stated properties of hyaluronic acid are dependent on the molecular weight, the solution concentration, and physiological pH. At low concentrations, the individual chains entangle and form a continuous network in solution, which gives the system interesting properties, such as pronounced viscoelasticity and pseudoplasticity that is unique for a water-soluble polymer at low concentration.

For example, the concentration of hyaluronic acid or salt thereof in the composition is from 0.05 %w/v to 0.5 %w/v or from 0.05 %w/v to 0.2 %w/v. The average molecular weight of the hyaluronic acid or salt thereof is from 500 kD to 5000 kD, or from 1000 kD to 3000 kD.

Alginate is an anionic biopolymers produced by a variety of microorganisms and marine algae. Alginate is a polysaccharide that comprises β-D-mannuronic acid units and α-L-guluronic acid units. Some alginate polymers are block copolymers with blocks of the guluronic acid (or salt) units alternating with blocks of the mannuronic acid (or salt) units as depicted in-part below.

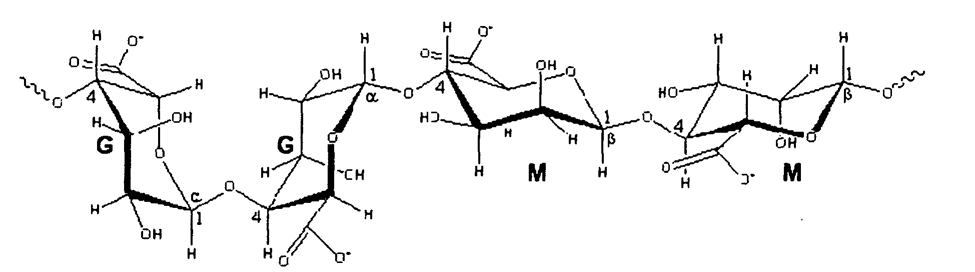

Some alginate molecules have single units of guluronic acid (or salt) alternating with single units of mannuronic acid (or salt). The ratio and distribution of the mannuronic and guluronic unit, along with the average molecular weight, affect the physical and chemical properties of the copolymer. See Haug, A. et al., Acta Chem. Scand., 183-90 (1966). Alginate polymers have viscoelastic rheological properties and other properties that make it suitable for some medical applications. See Klock, G. et al., "Biocompatibility of mannuronic acid-rich alginates," Biomaterials, Vol. 18, No. 10, 707-13 (1997). The use of alginate as a thickener for topical ophthalmic use is disclosed in U.S. Patent No. 6,528,465 and U.S. Patent Application Publication 2003/0232089. In U.S. Patent No. 5,776,445, alginate is used as a drug delivery agent that is topically applied to the eye. U.S. Patent Publication No. 2003/0232089 teaches a dry-eye formulation that contains two polymer ingredients including alginate.

The alginate used in the compositions will typically have a number average molecular weight from about 20 kDa to 2000 kDa, or from about 100 kDa to about 1000 kDa, for example about 325 kDa. The concentration of alginate is from about 0.01 wt.% to about 2.0 wt.%. More, typically, the concentration of alginate is a from about 0.1 wt.% to about 0.5 wt.%.

Chitin is a naturally occurring biopolymer found in the shells of crustaceans such as shrimp, crab, and lobster, and can be isolated from these shells using aqueous solutions that are highly acidic or highly basic. It is a linear polymer formed through β-(1,4) glycosidic linkage of the monomeric N-acetyl-D-glucosamine. The chitin obtained from such sources is not normally soluble in aqueous solutions at neutral pH so various chemical modifications have been adopted to enhance the solubility of chitin. For example, chitin can be deacetylated to obtain chitosan, which is relatively soluble in aqueous compositions.

Accordingly, the compositions can include contain one or more anionic chitosan derivatives that are soluble in aqueous solutions at a pH of from 6.5-8.5. The anionic chitosan derivatives have one or more anionic functional groups, such as sulfuryl chitosan, phosphoryl chitosan, carboxymethyl chitosan, dicarboxymethyl chitosan, and succinyl chitosan. A preferred chitosan derivative is carboxymethyl chitosan. The chitosan polymers can have an average number molecular weight ranging from 1 kD to 10,000 kD.

Some of the chitosan derivatives used in the compositions are commercially available (e.g., carboxymethyl chitosan is available from KoYo Chemical Co., LTD., Tokyo, Japan); or can be prepared by means of processes that have been described in the scientific literature [e.g., Ryoichi Senju and Satoshi Okimasu, Nippon Nogeikagaku Kaishi, vol. 23, 4324-37, (1950); Keisuke Kurita, J Synthetic Organic Chemistry Japan, vol. 42, 567-574, (1984); and Seiichi Tokura, Norio Nishi, Akihiro Tsutsumi, and Oyin Somorin, Polymer J, vol. 15, 485-489 (1983)].

Other types of anionic biopolymers that can be used in the compositions include carboxymethylcellulose and salts thereof, salts of carboxymethyl and carboxymethylhydroxyethyl starchs, and other glucoaminoglycans such as chondroitin sulfate, dermatan sulfate, heparin and heparin sulfate and keratin sulfates.

It is to be understood by those in the art that the compositions can include one or more of the anionic biopolymers described above such as a mixture of hyaluronic acid and alginate.

The compositions also include an antimicrobial component selected from certain quaternary ammonium compounds (including small molecules) and polymers and low and high molecular weight biguanides as defined in claim 7. For example, biguanides include the free bases or salts of alexidine, chlorhexidine, hexamethylene biguanides and their polymers, and combinations thereof. The salts of alexidine and chlorhexidine can be either organic or inorganic and include gluconates, nitrates, acetates, phosphates, sulfates, halides and the like.

In a preferred embodiment, the composition will include a polymeric biguanide known as poly(hexamethylene biguanide) (PHMB or PAPB) commercially available from Zeneca, Wilmington, DE under the trademark COSMOCil^{™} CQ. The PHMB is present in the compositions from 0.2 ppm to 5 ppm or from 0.5 ppm to 2 ppm.

One of the more common quaternary ammonium compounds is α-[4-tris(2-hydroxyethyl)-ammonium chloride-2-butenyl]poly[1-dimethyl ammonium chloride-2-butenyl]-ω-tris(2-hydroxyethyl) ammonium chloride, also referred to in the art as polyquaternium-1. The more common guaternary ammonium compounds are generally referred to in the art as "polyquaternium" disinfectants, and are identified by a particular number following the designation such as polyquaternium-1, polyquaternium-10 or polyquaternium-42. Polyquaternium-1 is present in the ophthalmic compositions from 0.5 ppm to 15 ppm.

Polyquaternium-42 is also one of the more preferred polyquaternium disinfectants, see, U.S. Patent No. 5,300,296. Polyquaternium-42 is present in the ophthalmic compositions from 5 ppm to 50 ppm.

It is to be understood by those in the art that the compositions can include one or more of the antimicrobial components described above. For example, in one embodiment, the ophthalmic compositions include polyquaternium-1 in combination with a biguanide antimicrobial component such as poly(hexamethylene biguanide). The polyquaternium-1 is present in relatively low concentrations, that is, from 0.5 ppm to 5 ppm, relative to the reported concentration of polyquaternium-1 in both Opti-Free® and Opti-Free®Replenish. Applicants believe that the polyquaternium-1 and the PHMB, in combination, may enhance the biocidal efficacy of the ophthalmic compositions.

### Contact Lens Care Compositions

The contact lens care solutions will very likely include a buffer system. By the terms "buffer" or "buffer system" is meant a compound that, usually in combination with at least one other compound, provides a buffering system in solution that exhibits buffering capacity, that is, the capacity to neutralize, within limits, either acids or bases (alkali) with relatively little or no change in the original pH. Generally, the buffering components are present from 0.05% to 2.5% (w/v) or from 0.1% to 1.5% (w/v).

The term "buffering capacity" is defined to mean the millimoles (mM) of strong acid or base (or respectively, hydrogen or hydroxide ions) required to change the pH by one unit when added to one liter (a standard unit) of the buffer solution. The buffer capacity will depend on the type and concentration of the buffer components. The buffer capacity is measured from a starting pH of 6 to 8, preferably from 7.4 to 8.4.

Borate buffers include, for example, boric acid and its salts, for example, sodium borate or potassium borate. Borate buffers also include compounds such as potassium tetraborate or potassium metaborate that produce borate acid or its salt in solutions. Borate buffers are known for enhancing the efficacy of certain polymeric biguanides. For example, U.S. Pat. No. 4,758,595 to Ogunbiyi et al. describes that a contact-lens solution containing PHMB can exhibit enhanced efficacy if combined with a borate buffer.

A phosphate buffer system preferably includes one or more monobasic phosphates, dibasic phosphates and the like. Particularly useful phosphate buffers are those selected from phosphate salts of alkali and/or alkaline earth metals. Examples of suitable phosphate buffers include one or more of sodium dibasic phosphate (Na₂HPO₄) sodium monobasic phosphate (NaH₂PO₄) and potassium monobasic phosphate (KH₂PO₄). The phosphate buffer components frequently are used in amounts from 0.01 % or to 0.5% (w/v), calculated as phosphate ion.

Other known buffer compounds can optionally be added to the lens care compositions, for example, citrates, citric acid, sodium bicarbonate, TRIS, and the like. Other ingredients in the solution, while having other functions, may also affect the buffer capacity, e.g., propylene glycol or glycerin.

A preferred buffer system is based upon boric acid/borate, a mono and/or dibasic phosphate salt/phosphoric acid or a combined boric/phosphate buffer system. For example a combined boric/phosphate buffer system can be formulated from a mixture of boric acid/sodium borate and a monobasic/dibasic phosphate. In a combined boric/phosphate buffer system, the phosphate buffer is used (in total) at a concentration of 0.004 to 0.2 M (Molar), preferably 0.04 to 0.1 M. The borate buffer (in total) is used at a concentration of 0.02 to 0.8 M, preferably 0.07 to 0.2 M.

The lens care solutions can also include an effective amount of a surfactant component, a viscosity inducing or thickening component, a chelating or sequestering component, or a tonicity component. The additional component or components can be selected from materials which are known to be useful in contact lens care solutions and are included in amounts effective to provide the desired functional characteristic.

Suitable surfactants can be cationic or nonionic, and are typically present (individually or in combination) in amounts up to 2 %w/v. One preferred surfactant class are the nonionic surfactants. The surfactant should be soluble in the lens care solution and nonirritating to eye tissues. Many nonionic surfactants comprise one or more chains or polymeric components having oxyalkylene (--O--R--) repeats units wherein R has 2 to 6 carbon atoms. Preferred non-ionic surfactants comprise block polymers of two or more different kinds of oxyalkylene repeat units, which ratio of different repeat units determines the HLB of the surfactant. Satisfactory non-ionic surfactants include polyethylene glycol esters of fatty acids, e.g. coconut, polysorbate, polyoxyethylene or polyoxypropylene ethers of higher alkanes (C₁₂-C₁₈). Examples of this class include polysorbate 20 (available under the trademark Tween® 20), polyoxyethylene (23) lauryl ether (Brij® 35), polyoxyethyene (40) stearate (Myrj®52), polyoxyethylene (25) propylene glycol stearate (Atlas® G 2612). Still another preferred surfactant is tyloxapol.

A particular non-ionic surfactant consisting of a poly(oxypropylene)-poly(oxyethylene) adduct of ethylene diamine having a molecular weight from about 6,000 to about 24,000 daltons wherein at least 40 weight percent of said adduct is poly(oxyethylene) has been found to be particularly advantageous for use in cleaning and conditioning both soft and hard contact lenses. The CTFA Cosmetic Ingredient Dictionary's adopted name for this group of surfactants is poloxamine. Such surfactants are available from BASF Wyandotte Corp., Wyandotte, Mich., under Tetronic®. Particularly good results are obtained with poloxamine 1107 or poloxamine 1304. The foregoing poly(oxyethylene) poly(oxypropylene) block polymer surfactants will generally be present in a total amount from 0.0 to 2 %w/v, from 0. to 1 % w/v, or from 0.2 to 0.8 %w/v An analogous of series of surfactants, for use in the lens care compositions, is the poloxamer series which is a poly(oxyethylene) poly(oxypropylene) block polymers available under Pluronic® (commercially available form BASF). In accordance with one embodiment of a lens care composition the poly(oxyethylene)-poly(oxypropylene) block copolymers will have molecular weights from 2500 to 13,000 daltons or from 6000 to about 12,000 daltons. Specific examples of surfactants which are satisfactory include: poloxamer 108, poloxamer 188, poloxamer 237, poloxamer 238, poloxamer 288 and poloxamer 407. Particularly good results are obtained with poloxamer 237 or poloxamer 407. The foregoing poly(oxyethylene) poly(oxypropylene) block polymer surfactants will generally be present in a total amount from 0.0 to 2 %w/v, from 0. to 1% w/v, or from 0.2 to 0.8 %w/v.

The compositions can also include an amphoteric surfactant. Suitable amphoteric surfactants include betaine and sulphobetaine surfactants, derivatives thereof. The betaine or sulphobetaine surfactants are believed to contribute to the disinfecting properties of the compositions by increasing the permeability of the bacterial cell wall, thus allowing an antimicrobial agent to enter the cell.

The amphoteric surfactants of general formula I are surface-active compounds with both acidic and alkaline properties. The amphoteric surfactants of general formula I include a class of compounds known as betaines. The betaines are characterized by a fully quaternized nitrogen atom and do not exhibit anionic properties in alkaline solutions, which means that betaines are present only as zwitterions at near neutral pH.

All betaines are characterized by a fully quaternized nitrogen. In alkyl betaines, one of the alkyl groups of the quaternized nitrogen is an alkyl chain with eight to thirty carbon atoms. One class of betaines is the sulfobetaines or hydroxysulfobetaines in which the carboxylic group of alkyl betaine is replaced by sulfonate. In hydroxysulfobetaines a hydroxy-group is positioned on one of the alkylene carbons that extend from the quaternized nitrogen to the sulfonate. In alkylamido betaines, an amide group is inserted as a link between the hydrophobic C₈-C₃₀alkyl chain and the quaternized nitrogen.

Accordingly, the ophthalmic compositions may comprise: ppm to 50 ppm of a cationic antimicrobial component selected from the group consisting of biguanides, polymeric biguanides, quatemium ammonium compounds and any one mixture thereof; 0.005 wt.% to 2 wt.% of an anionic biopolymer; and 0.01 wt.% to 2 wt.% of an amphoteric surfactant of general formula I 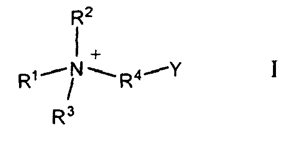 wherein R¹ is R or (CH₂)ₙ-NHC(O)R, wherein R is a C₈-C₃₀alkyl optionally substituted with hydroxyl and n is 2, 3 or 4; R² and R³ are each independently selected from the group consisting of hydrogen and C₁-C₄alkyl; R⁴ is a C₂-C₈alkylene optionally substituted with hydroxyl; and Y is CO₂⁻ or SO₃⁻.

In one embodiment, the anioinic biopolymer is hyaluronic acid, which is present from 0.002 wt.% to 0.04 wt.%, and the cationic, antimicrobial component is poly(hexamethylene biguanide). Accordingly, one of the more preferred compositions comprises 0.5 ppm to 3.0 ppm of poly(hexamethylene biguanide); 0.002 wt.% to 0.04 wt.% hyaluronic acid; and 0.01 wt.% to 2 wt.% of an amphoteric surfactant of general formula I 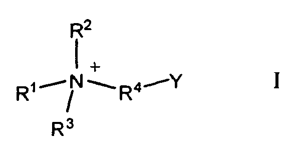 wherein R¹ is R or -CH₂)ₙ-NHC(O)R, wherein R is a C₈-C₃₀alkyl optionally substituted with hydroxyl and n is 2, 3 or 4; R² and R³ are each independently selected from the group consisting of hydrogen and C₁-C₄alkyl; R⁴ is a C₂-C₈alkylene optionally substituted with hydroxyl; and Y is CO₂⁻ or SO₃⁻. In many embodiments, the amphoteric surfactant of general formula I is a sulfobetaine of general formula II 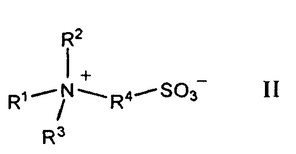 wherein R¹ is a C₈-C₃₀alkyl; R² and R³ are each independently selected from a C₁-C₄alkyl; and R⁴ is a C₂-C₈alkylene.

Certain sulfobetaines of general formula II are more preferred than others. For example, Zwitergent®3-10 available from Calbiochem Company, is a sulfobetaine of general formula I wherein R¹ is a straight, saturated alkyl with ten (10) carbons, R² and R³ are each methyl and R⁴ is -CH₂CH₂CH₂- (three carbons, (3)). Other sulfobetaines that can be used in the ophthalmic compositions include the corresponding Zwitergent®3-08 (R¹ is a is a straight, saturated alkyl with eight carbons), Zwitergent®3-12 (R¹ is a is a straight, saturated alkyl with twelve carbons), Zwitergent®3-14 (R¹ is a is a straight, saturated alkyl with fourteen carbons) and Zwitergent®3-16 (R¹ is a is a straight, saturated alkyl with sixteen carbons). Accordingly, some of the more preferred the ophthalmic composition will include a sulfobetaine of general formula II wherein R' is a C₈-C₁₆alkyl and R² and R³ is methyl.

In another embodiment, the amphoteric surfactant of general formula I is a hydroxysulfobetaine of general formula III 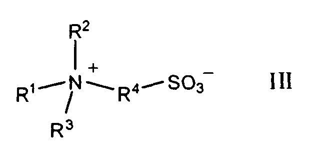 wherein R¹ is a C₈-C₃₀alkyl substituted with at least one hydroxyl; R² and R³ are each independently selected from a C₁-C₄alkyl; and R⁴ is a C₂-C₈alkylene substituted with at least one hydroxyl.

In another embodiment, the amphoteric surfactant is an alkylamido betaine of general formula IV 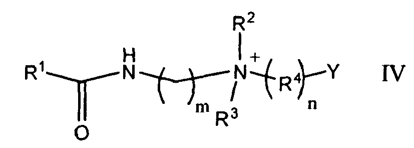 wherein R¹ is a C₈-C₃₀alkyl, and m and n are independently selected from 2, 3, 4 or 5; R² and R³ are each independently selected from a C₁-C₄alkyl optionally substituted with hydroxyl; R⁴ is a C₂-C₈alkylene optionally substituted with hydroxyl; and Y is CO₂⁻ or SO₃⁻. The most common alkylamido betaines are alkylamidopropyl betaines, e.g., cocoamidopropyl dimethyl betaine and lauroyl amidopropyl dimethyl betaine.

The lens care solutions can also include a phosphonic acid, or its physiologically compatible salt, that is represented by the following formula: 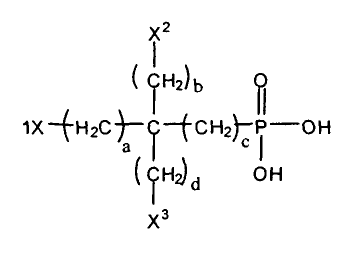 wherein each of a, b, c, and d are independently selected from integers from 0 to 4, preferably 0 or 1; X¹ is a phosphonic acid group (i.e., P(OH)₂O), hydroxy, amine or hydrogen; and X² and X³ are independently selected from the group consisting of halogen, hydroxy, amine, carboxy, alkylcarbonyl, alkoxycarbonyl, or substituted or unsubstituted phenyl, and methyl. Exemplary substituents on the phenyl are halogen, hydroxy, amine, carboxy and/or alkyl groups. A particularly preferred species is that wherein a, b, c, and d in are zero, specifically the tetrasodium salt of 1-hydroxyethylidene-1,1-diphosphonic acid, also referred to as tetrasodium etidronate, commercially available from Monsanto Company as DeQuest® 2016 diphosphonic acid sodium salt or phosphonate.

The lens care solutions can include dexpanthenol, which is an alcohol of pantothenic acid, also called Provitamin B5, D-pantothenyl alcohol or D-panthenol. It has been stated that dexpanthenol may play a role in stabilizing the lachrymal film at the eye surface following placement of a contact lens on the eye. Dexpanthenol is preferably present in the solution in an amount from 0.2 to 5 % /v, from 0.5 to 3 %w/v, or from 1 to 2 %w/v.

The contact lens care solutions can also include a sugar alcohol such as sorbitol or xylitol. Typically, dexpanthenol is used in combination with the sugar alcohol. The sugar alcohol is present in the lens care compositions in an amount from 0.4 to 5 %w/v or from 0.8 to 3 %w/v.

The lens care solutions can also include one or more neutral or basic amino acids. The neutral amino acids include: the alkyl-group-containing amino acids such as alanine, isoleucine, valine, leucine and proline; hydroxyl-group-containing amino acids such as serine, threonine and 4-hydroxyproline; thio-group-containing amino acids such as cysteine, methionine and asparagine. Examples of the basic amino acid include lysine, histidine and arginine. The one or more neutral or basic amino acids are present in the compositions at a total concentration of from 0.1 to 3 %w/v.

The lens care solutions can also include glycolic acid, asparatic acid or any mixture of the two at a total concentration of from 0.001% to 4% (w/v) or from 0.01% to 2.0% (w/v). In addition, the combined use of one or more amino acids and glycolic acid and/or asparatic acid can lead to a reduction in the change of the size of the contact lens due to swelling and shrinkage following placement in the lens solution.

The ophthalmic compositions can also include any monoterpene, sesquiterpene and/or diterpene or derivatives thereof. Acyclic, monocyclic and/or bicyclic mono-, sesqui- and/or diterpenes, and those with higher numbers of rings, can be used. A "derivative" of a terpene as used herein shall be understood to mean a terpene hydrocarbon having one or more functional groups such as terpene alcohols, terpene ethers, terpene esters, terpene aldehydes, terpene ketones and the like and combinations thereof. Here, both the trans and also the cis isomers are suitable. The terpenes as well as the terpene moiety in the derivative can contain from 6 to about 100 carbon atoms and preferably from about 10 to about 25 carbon atoms.

The ophthalmic composition can also include an at least one epithelium cell stabilizer selected from the group consisting of diglycine, glycine, triglycine, tetraglycine and pentaglycine. The epithelium cell stabilizer is generally present in the composition at a concentration of from 0.01 % w/w to a 4 % w/w, for instance 0.1 % w/w to 2.5 % w/w or 0.1 % w/w to 1 % w/w.

The normal conjunctiva and cornea are protected by a triple-layered tear film comprising an outer oily layer from the meibomian glands, an aqueous layer from lacrimal glands and an inner layer of mucus, derived mainly from conjunctival goblet cells. A stable tear film can be critical to prevent pathogenic microorganism invasion. Microorganism invasion can be facilitated by an epithelial defect, unstable tear film, or contaminated contact lenses. A stable preocular tear film depends on many factors, including the correct quantity and quality of various components of the tears and the integrity of the corneal epithelium. Environmental pollution, smoking and frequent use of eye drops can cause denaturization of tear proteins such as lysozyme and lactoferrin. The denatured tear proteins can cause destabilization of tear film, staining, loss of tight junction, and dry eye. The epithelium cell stabilizer is used to stabilize the tear proteins, which in turn, helps to stabilize the tear film.

The lens care solutions can also include one or more comfort or cushioning components, in addition to the carboxyl-modified fructan. The comfort component can enhance and/or prolong the cleaning and wetting activity of the surfactant component and/or condition the lens surface rendering it more hydrophilic (less lipophilic) and/or to act as a demulcent on the eye. The comfort component is believed to cushion the impact on the eye surface during placement of the lens and serves also to alleviate eye irritation.

Suitable comfort components include, but are not limited to, water soluble natural gums, cellulose-derived polymers and the like. Useful natural gums include guar gum, gum tragacanth and the like. Useful cellulose-derived comfort components include cellulose-derived polymers, such as hydroxypropyl cellulose, hydroxypropylmethyl cellulose, carboxymethyl cellulose, methyl cellulose, hydroxyethyl cellulose and the like. A very useful comfort component is hydroxypropylmethyl cellulose (HPMC). Some non-cellulose comfort components include hydroxypropyl guar, propylene glycol or glycerin. The comfort components are typically present in the solution from 0.01% to 1% (w/v).

One preferred comfort agent that is believed to maintain a hydrated corneal surface is polyvinylpyrrolidone (PVP). PVP is a linear homopolymer or essentially a linear homopolymer comprising at least 90% repeat units derived from 1-vinyl-2-pyrrolidone monomer, the remainder of the monomer composition can include neutral monomer, e.g., vinyl or acrylates. Other synonyms for PVP include povidone, polyvidone, 1-vinyl-2-pyrolidinone, and 1-ethenyl-2-pyrolionone (CAS registry number 9003-39-8). The PVP will preferably have a weight average molecular weight from 10,000 to 250,000 or from 30,000 to 100,000. Such materials are sold by various companies, including ISP Technologies, Inc. under the trademark PLASDONE®K-29/32, from BASF under the trademark KOLLIDON®, for example, KOLLIDON® K-30 or K-90. It is also preferred that one use pharmaceutical grade PVP.

The lens care solutions can also include one or more chelating components to assist in the removal of lipid and protein deposits from the lens surface following daily use. Typically, the ophthalmic compositions will include relatively low amounts, e.g., from 0.005% to 0.05 % (w/v) of ethylenediaminetetraacetic acid (EDTA) or the corresponding metal salts thereof such as the disodium salt, Na₂EDTA.

One possible alternative to the chelator Na₂EDTA or a possible combination with Na₂EDTA, is a disuccinate of formula IV below or a corresponding salt thereof; 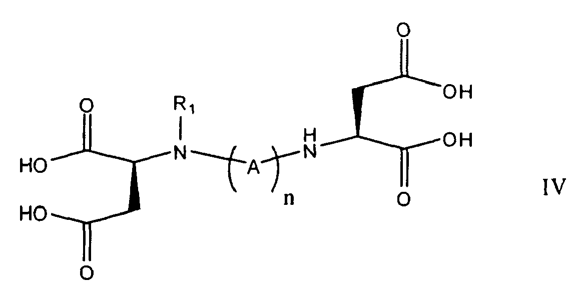 wherein R₁ is selected from hydrogen, alkyl or -C(O)alkyl, the alkyl having one to twelve carbons and optionally one or more oxygen atoms, A is a methylene group or an oxyalkylene group, and n is from 2 to 8. In one embodiment, the disuccinate is S,S-ethylenediamine disuccinate (S,S-EDDS) or a corresponding salt thereof. One commercial source of S,S-EDDS is represented by Octaquest® E30, which is commercially available from Octel. The chemical structure of the trisodium salt of S,S-EDDS is shown below. The salts can also include the alkaline earth metals such as calcium or magnesium. The zinc or silver salt of the disuccinate can also be used in the ophthalmic compositions.

Still another class of chelators include alkyl ethylenediaminetriacetates such as nonayl ethylenediaminetriacetate. See, U.S. Patent No. 6,995,123 for a more complete description of such agents.

The lens care solutions will typically include an effective amount of a tonicity adjusting component. Among the suitable tonicity adjusting components that can be used are those conventionally used in contact lens care products such as various inorganic salts. Sodium chloride and/or potassium chloride and the like are very useful tonicity components. The amount of tonicity adjusting component is effective to provide the desired degree of tonicity to the solution.

The lens care solutions will typically have an osmolality in the range of at least about 200 mOsmol/kg for example, about 300 or about 350 to about 400 mOsmol/kg. The lens care solutions are substantially isotonic or hypertonic (for example, slightly hypertonic) and are ophthalmically acceptable.

The ophthalmic compositions herein can be formulated by combining the essential and preferred components in the requisite amounts in any suitable order and in any conventional manner for formulations of this type. Generally such compositions can be prepared by adding the active or functional components to deionized water under conditions which dissolve or disperse those components in the aqueous compositions.

The compositions are used as a disinfecting solution, a preservative solution or packaging solution for contact lenses including (1) hard lenses formed from materials prepared by polymerization of acrylic esters such as polymethyl methacrylate (PMMA), (2) rigid gas permeable (RGP) lenses formed from silicone acrylates and fluorosilicone methacrylates, (3) soft, hydrogel lenses, and (4) non-hydrogel elastomer lenses.

Accordingly, a method for preserving, disinfecting or cleaning contact lenses may be performed. In general, such a method comprises contacting the lenses with an ophthalmic composition. Although such contacting may be accomplished by simply soaking a lens in the ophthalmic composition, greater preserving, disinfecting or cleaning may possibly be achieved if a few drops of the composition are initially placed on each side of the lens, and the lens is rubbed for a period of time, for example, approximately 20 seconds. The lens can then be subsequently immersed within several milliliters of the subject composition. Preferably, the lens is permitted to soak in the composition for at least four hours. Furthermore, the lens is preferably rinsed with fresh composition after any rubbing step and again after being immersed within the composition. The lenses can then be removed from the composition, rinsed with the same or a different liquid, for example, a preserved isotonic saline solution and placed on the eye.

As an example, soft hydrogel contact lenses are made of a hydrogel polymeric material, a hydrogel being defined as a crosslinked polymeric system containing water in an equilibrium state. In general, hydrogels exhibit excellent biocompatibility properties, i.e., the property of being biologically or biochemically compatible by not producing a toxic, injurious or immunological response in a living tissue. Representative conventional hydrogel contact lens materials are made by polymerizing a monomer mixture comprising at least one hydrophilic monomer, such as (meth)acrylic acid, 2-hydroxyethyl methacrylate (HEMA), glyceryl methacrylate, N,N-dimethacrylamide, and N-vinylpyrrolidone (NVP). In the case of silicone hydrogels, the monomer mixture from which the copolymer is prepared further includes a siloxy-containing monomer, in addition to the hydrophilic monomer. Generally, the monomer mixture will include a crosslinking monomer, i.e., a monomer having at least two polymerizable radicals, such as ethylene glycol dimethacrylate, tetraethylene glycol dimethacrylate, and methacryloxyethyl vinylcarbonate. Alternatively, either the siloxy-containing monomer or the hydrophilic monomer may function as a crosslinking agent.

The ophthalmic compositions can also be used to treat a patient diagnosed with dry eye. The patient would administer the ophthalmic composition to the eye, eye lid or to the skin surrounding the eye. The compositions are thus useful for relieving eye irritation or dryness and providing lubrication for the eyes, irrespective of whether contact lenses are present in the eyes of the patient.

The ophthalmic compositions can be formulated to function as artificial tears and can be used, as needed, for the temporary relief of eye irritation or discomfort. For example, many people suffer from temporary or chronic eye conditions in which the eye's tear system fails to provide adequate tear volume or tear film stability necessary to remove irritating environmental contaminants such as dust, pollen, or the like. In persons suffering from chronic dry eye, the film on the eye tends to becomes discontinuous. The ophthalmic compositions can be used to treat the above conditions.

### Example Compositions

In the example compositions below, certain chemical components are identified by the following abbreviations. The amounts of each recited component are in wt.% except those indicated as ppm.
EDTA: EthylenediamineTetraacetic Acid
PHMB: Poly(hexamethylene biguanide)
Dequest^{®}: is a 30 wt.% aqueous solution of hydroxyalkylphosphonate.
Dequest^{®}PB: is an aqueous solution of carboxymethylinulin.
Tetronic^{®} 1107: a surfactant, commercially available from BASF.
Pluronic^{®} P123: a surfactant, commercially available from BASF.
Pluronic^{®} F127: a surfactant, commercially available from BASF.

**TABLE 1**

| **Example** | **1** | **2** | **3** | **4** | **5** |
|---|---|---|---|---|---|
| Boric Acid | 0.85 | 0.85 | 0.85 | 0.85 | 0.85 |
| Na phosphate monobasic | 0.31 | 0.31 | 0.31 | 0.31 | 0.31 |
| Na phosphate dibasic | 0.15 | 0.15 | 0.15 | 0.15 | 0.15 |
| Citric acid | - | - | - | - | 0.15 |
| Dequest^{®}PB | 0.03 | 0.03 | 0.03 | 0.03 | 0.03 |
| Tetronic^{®} 1107 | 1.0 | 1.0 | 1.0 | 1.0 | 1.0 |
| Geraniol | 0.0001 | - | - | | |
| α-Terpineol | - | 0.0001 | - | 0.0001 | |
| 1-terpene-4-ol | - | - | 0.0001 | | 0.0001 |
| Polyquaternium-1 | - | - | - | 0.008 | 0.008 |
| PHMB | 0.0001 | 0.0001 | 0.0001 | - | - |
| Purified water | Q.S. to 100% | Q.S. to 100% | Q.S. to 100% | Q.S. to 100% | Q.S. to 100% |

**TABLE 2**

| **Example** | **Comp. 1** | **6** | **Comp. 2** | **7** |
|---|---|---|---|---|
| Boric Acid | 0.85 | 0.85 | 0.85 | 0.85 |
| Sodium Phosphate monobasic | 0.15 | 0.15 | 0.15 | 0.15 |
| Sodium Phosphate dibasic | 0.31 | 0.31 | 0.31 | 0.31 |
| Sodium Chloride | 0.19 | 0.19 | 0.19 | 0.19 |
| Citric acid | 0.15 | 0.15 | - | - |
| Dequest (phosphonate) | 0.03 | - | 0.03 | - |
| Dequest^{®}PB (CMI) | - | 0.03 | - | 0.03 |
| Tetronic^{®} 1107 | 1.00 | 1.00 | 1.00 | 1.00 |
| Polyquertemium-1 | 0.0008 | 0.0008 | - | - |
| PHMB | - | - | 0.0001 | 0.0001 |
| Purified water | Q.S. to 100% | Q.S. to 100% | Q.S. to 100% | Q.S. to 100% |

**Table 3.**

| **Example** | **8** | **9** | **10** | **11** | **12** |
|---|---|---|---|---|---|
| sodium citrate | 0.26 | - | 0.6 | - | - |
| sodium chloride | 0.35 | 0.1 | 0.1 | 0.3 | 0.25 |
| sodium borate | 0.15 | - | 0.60 | 0.33 | - |
| boric acid | 0.45 | - | - | 0.45 | - |
| Na phosphate dibasic | - | 0.45 | - | - | 0.45 |
| diglycine | - | - | - | 0.26 | - |
| propylene glycol | - | - | 1.00 | 0.6 | 0.6 |
| HPMC E15LV | 0.15 | - | - | 0.15 | 0.15 |
| Dequest^{®}PB (CMI) | 0.05 | 0.05 | 0.05 | 0.05 | 0.05 |
| Tetronic®1304 | 0.1 | - | 0.10 | 0.1 | - |
| Pluronic®F127 | - | 0.2 | - | - | 0.35 |
| sorbitol | - | 1.6 | - | - | |
| dexpanthenol | - | 2.0 | - | - | - |
| tromethamine | - | 0.35 | - | - | - |
| Na₂EDTA | 0.1 | 0.05 | - | - | 0.08 |
| nonanoyl EDTA | - | - | 0.20 | 0.2 | - |
| polyquaternium-1 (ppm) | 10 | - | 10 | 10 | - |
| PHMB (ppm) | - | 1.2 | - | - | 1.2 |

**Table 4.**

| **Example** | **13** | **14** |
|---|---|---|
| sodium chloride | 0.4 | 0.15 |
| sodium borate | 0.18 | 0.18 |
| boric acid | 0.45 | 0.45 |
| Na phosphate dibasic | - | 0.3 |
| Na phosphate monobasic | - | 0.02 |
| propylene glycol | - | 0.3 |
| HPMC E15LV | 0.15 | 0.15 |
| Dequest^{®}PB (CMI) | 0.05 | 0.05 |
| Tetronic®1107 | 0.2 | 0.2 |
| Na₂EDTA | 0.1 | 0.08 |
| polyquaternium-1 (ppm) | 5 | 5 |
| PHMB (ppm) | 0.8 | 0.8 |

## Claims

1. The use of an ophthalmic composition comprising a carboxyl-modified fructan or a salt thereof in an eye care product, or a contact lens care product selected from the group consisting of contact lens rewet drops, contact lens packaging solution and contact lens disinfecting solution.

2. The use of the composition as in claim 1 wherein the composition further comprises a cationic antimicrobial component selected from the group consisting of α-[4-tris(2-hydroxyethyl)ammonium chloride-2-butenyl]poly[1-dimethylammonium chloride-2-butenyl]-ω-tris(2-hydroxyethyl)ammonium chloride, benzalkonium halides, alexidine and salts thereof, hexamethylene biguanides and salts thereof and their polymers, and mixtures thereof.

3. The use of the composition as in claim 2 wherein the cationic antimicrobial component is selected from the group consisting of poly(hexamethylene biguanide), which is present from 0.2 ppm to 2 ppm, α-[4-tris(2-hydroxyethyl)ammonium chloride-2-butenyl]poly[1-dimethylammonium chloride-2-butenyl]-ω-tris(2-hydroxyethyl) ammonium chloride, which is present from 2 ppm to 15 ppm, and any mixture thereof.

4. The use of the composition as in any one of claims 1 to 3 wherein the carboxyl-modified fructan or the salt thereof is a carboxyalkylinulin or a salt thereof.

5. The use of the composition as in claim 4 wherein the carboxyalkylinulin is selected from the group consisting of carboxymethylinulin, carboxyethylinulin or a salt of each thereof.

6. The use of the composition as in claims 4 or 5 wherein the carboxyalkylinulin includes 0.3 to 3 carboxylgroups per anhydrofructose unit.

7. An ophthalmic composition comprising:
a carboxyl-modified fructan or a salt thereof; and
a cationic antimicrobial component selected from the group consisting of α-[4-tris(2-hydroxyethyl)ammonium chloride-2-butenyl]poly[1-dimethylammonium chloride-2-butenyl]-ω-tris(2-hydroxyethyl)ammonium chloride, benzalkonium halides, alexidine and salts thereof, hexamethylene biguanides and salts thereof and their polymers, and mixtures thereof.

8. The composition of claim 7 wherein the carboxyl-modified fructan includes 0.3 to 3 carboxylgroups per anhydrofructose unit.

9. The composition of claims 7 or 8 wherein the cationic antimicrobial component is selected from the group consisting of poly(hexamethylene biguanide), which is present from 0.2 ppm to 2 ppm, α-[4-tris(2-hydroxyethyl)ammonium chloride-2-butenyl]poly[1-dimethylammonium chloride-2-butenyl]-ω-tris(2-hydroxyethyl) ammonium chloride, which is present from 2 ppm to 15 ppm, and any mixture thereof.

10. The composition of claims 7 to 9 wherein the carboxyl-modified fructan or the salt thereof is a carboxyalkylinulin or a salt thereof.

11. The composition of any one of claims 7 to 10 further comprising one or more biopolymers and any derivative thereof selected from the group consisting of hyaluronic acid and alginate.

12. The composition of any one of claims 7 to 11 further comprising dexpanthenol, sorbitol or any mixture thereof.

13. The composition of any one of claims 7 to 12 further comprising hydroxypropylmethyl cellulose, propylene glycol or hydroxypropyl guar.

14. The composition of any one of claims 7 to 13 wherein the composition further comprises a betaine or a sulphobetaine surfactant of formula I 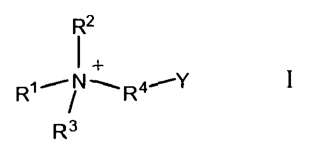 wherein R¹ is R or -(CH₂)ₙ-NHC(O)R, wherein R is a C₈-C₃₀alkyl optionally substituted with hydroxyl and n is 2, 3 or 4; R² and R³ are each independently selected from the group consisting of hydrogen and C₁-C₄alkyl; R⁴ is a C₂-C₈alkylene optionally substituted with hydroxyl; and Y is CO₂⁻ or SO₃⁻.

15. The composition of claim 14 wherein R¹ is R; R² and R³ are each independently selected from a C₁-C₂alkyl; R⁴ is a C₂-C₄alkylene and Y is SO₃⁻.

## Patentansprüche

1. Die Verwendung einer ophthalmischen Zusammensetzung die ein Carboxyl-modifiziertes Fruktan oder ein Salz davon in einem Augenpflegeprodukt oder einem Kontaktlinsenpflegeprodukt, ausgewählt aus der Gruppe, die aus Kontaktlinsenwiederbefeuchtungstropfen, Kontaktlinsenverpackungslösungen und Kontaktlinsendesinfektionslösungen besteht, umfasst.

2. Die Verwendung einer ophthalmischen Zusammensetzung gemäß Anspruch 1, wobei die Zusammensetzung zusätzlich eine kationische antimikrobielle Komponente, ausgewählt aus einer Gruppe, die aus α-[4-Tris(2-hydroxyethyl)ammoniumchlorid-2-butenyl]poly[1-dimethylammoniumchlorid-2-butenyl]-ω-tris(2-hydroxyethyl)ammoniumchlorid, Benzalkoniumhaliden, Alexidin und Salzen davon, Hexamethylenbiguaniden und Salzen davon und deren Polymeren, und Mischungen davon besteht, umfasst.

3. Die Verwendung einer ophthalmischen Zusammensetzung gemäß Anspruch 2, wobei die eine kationische antimikrobielle Komponente aus einer Gruppe ausgewählt wird, die aus Poly(hexamethylenbiguanidin), das in einer Menge von 0,2 ppm bis 2 ppm vorhanden ist, α-[4-Tris(2-hydroxyethyl)ammoniumchlorid-2-butenyl]poly[1-dimethylammonium-chlorid-2-butenyl]-ω-tris(2-hydroxyethyl)ammoniumchlorid, das in einer Menge von 2 ppm bis 15 ppm vorhanden ist und allen Mischungen daraus, besteht.

4. Die Verwendung einer Zusammensetzung gemäß einem der Ansprüche 1 bis 3, wobei das Carboxyl-modifizierte Fruktan oder Salz davon ein Carboxyalkylinulin oder Salz davon ist.

5. Die Verwendung einer Zusammensetzung gemäß Anspruch 4, wobei das Carboxyalkylinulin aus einer Gruppe ausgewählt wird, die aus Carboxymethylinulin, Carboxyethylinulin oder einem Salz der vorgenannten besteht.

6. Die Verwendung der Zusammensetzung gemäß Anspruch 4 oder 5, wobei das Carboxyalkylinulin 0,3 bis 3 Carboxylgruppen pro Anhydrofruktoseeinheit beinhaltet.

7. Eine ophthalmische Zusammensetzung die:
ein Carboxyl-modifiziertes Fruktan oder Salz davon; und
eine kationische antimikrobielle Komponente, ausgewählt aus der Gruppe, die aus α-[4-Tris(2-hydroxyethyl)ammoniumchlorid-2-butenyl]poly[ 1-dimethylammoniumchlorid-2-butenyl]-ω-tris(2-hydroxyethyl)ammoniumchlorid, Benzalkoniumhaliden, Alexidin und Salzen davon, Hexamethylenbiguaniden und Salzen davon und deren Polymeren, und Mischungen davon besteht, umfasst.

8. Die Zusammensetzung gemäß Anspruch 7, wobei das Carboxyl-modifizierte Fruktan 0,3 bis 3 Carboxylgruppen pro Anhydrofruktoseeinheit enthält.

9. Die Zusammensetzung gemäß Anspruch 7 oder 8, wobei die kationische antimikrobielle Komponente aus einer Gruppe ausgewählt wird, die aus Poly(hexamethylen-biguanidin), das in einer Menge von 0,2 ppm bis 2 ppm vorhanden ist, α-[4-Tris(2-hydroxyethyl)ammoniumchlorid-2-butenyl]poly[1-dimethylammoniumchlorid-2-butenyl]-ω-tris(2-hydroxyethyl)ammoniumchlorid, das in einer Menge von 2 ppm bis 15 ppm vorhanden ist und jeglichen Mischungen daraus, besteht.

10. Die Zusammensetzung gemäß Anspruch 7 bis 9, wobei das Carboxyl-modifizierte Fruktan oder die Salze davon Carboxyalkylinulin oder Salze davon sind.

11. Die Zusammensetzung gemäß einem der Ansprüche 7 bis 10, zusätzlich ein oder mehrere Biopolymere und jegliche Derivate davon, ausgewählt aus der Gruppe, die aus Hyaluronsäure und Alginat besteht, umfassend.

12. Die Zusammensetzung gemäß einem der Ansprüche 7 bis 11, zusätzlich Dexpanthenol, Sorbitol oder jegliche Mischung daraus umfassend.

13. Die Zusammensetzung gemäß einem der Ansprüche 7 bis 12, zusätzlich Hydroxypropylmethylcellulose, Propylenglykol oder Hydroxypropylguar umfassend.

14. Die Zusammensetzung gemäß eines jeden Anspruches 7 bis 13, wobei die Zusammensetzung zusätzlich einen Betain- oder Sulphobetainsurfactanten der Formel I umfasst, 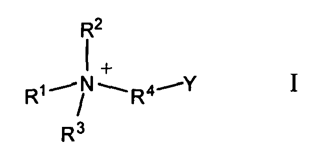 worin R¹ R oder -(CH₂)ₙ-NHC(O)R, worin R eine C₈-C₃₀ Alkylkette, optional mit Hydroxylgruppen substituiert, und n 2, 3 oder 4 ist; R² und R³ sind unabhängig voneinander aus einer Gruppe ausgewählt, die aus Wasserstoff und C₁-C₄ Alkylketten besteht; R⁴ ist eine C₂-C₈ Alkylkette, optional mit Hydroxylgruppen substituiert; und Y ist CO₂ oder SO₃⁻.

15. Die Zusammensetzung gemäß Anspruch 14, worin R¹ R ist; R² und R³ unabhängig voneinander eine C₁-C₂ Alkylkette sind; R⁴ ist eine C₂-C₄ Alkylkette und Y ist SO₃⁻.

## Revendications

1. Utilisation d'une composition ophtalmique comprenant un fructane modifié par carboxyle ou un sel de celui-ci dans un produit de soin pour les yeux, ou un produit de soin pour verres de contact choisi dans le groupe constitué par les gouttes d'humidification de verres de contact, une solution de conditionnement de verres de contact et une solution de désinfection de verres de contact.

2. Utilisation de la composition selon la revendication 1, dans laquelle la composition comprend en outre un composant antimicrobien cationique choisi dans le groupe constitué par le chlorure d'α-[chlorure de 4-tris(2-hydroxyéthyl)ammonium-2-butényl]poly[chlorure de 1-diméthylammonium-2-butényl]-ω-tris(2-hydroxyéthyl)ammonium, les halogénures de benzalkonium, l'aléxidine et leurs sels, les hexaméthylène biguanides et leurs sels, et leurs polymères, et leurs mélanges.

3. Utilisation de la composition selon la revendication 2, dans laquelle le composant antimicrobien cationique est choisi dans le groupe constitué par le poly(hexaméthylène biguanine), qui est présent de 0,2 ppm à 2 ppm, le chlorure d'α-[chlorure de 4-tris(2-hydroxyéthyl)ammonium-2-butényl]poly[chlorure de 1-diméthylammonium-2-butényl]-ω-tris(2-hydroxyéthyl)ammonium, qui est présent de 2 ppm à 15 ppm, et l'un quelconque de leurs mélanges.

4. Utilisation de la composition selon l'une quelconque des revendications 1 à 3, dans laquelle le fructane modifié par carboxyle ou son sel est une carboxyalkylinuline ou un de ses sels.

5. Utilisation de la composition selon la revendication 4, dans laquelle la carboxyalkylinuline est choisie dans le groupe constitué par la carboxyméthylinuline, la carboxyéthylinuline ou un sel de chacune d'entre elles.

6. Utilisation de la composition selon la revendication 4 ou 5, dans laquelle la carboxyalkylinuline comprend 0,3 à 3 groupes carboxyle par motif anhydrofructose.

7. Composition ophtalmique comprenant:
un fructane modifié par carboxyle ou un de ses sels; et
un composant antimicrobien cationique choisi dans le groupe constitué par le chlorure d'α-[chlorure de 4-tris(2-hydroxyéthyl)ammonium-2-butényl]poly[chlorure de 1-diméthylammonium-2-butényl]-ω-tris(2-hydroxyéthyl)ammonium, les halogénures de benzalkonium, l'aléxidine et leurs sels, les hexaméthylène biguanides et leurs sels, et leurs polymères, et leurs mélanges.

8. Composition selon la revendication 7, dans laquelle le fructane modifié par carboxyle comprend 0,3 à 3 groupes carboxyle par motif anhydrofructose.

9. Composition selon les revendications 7 ou 8, dans laquelle le composant antimicrobien cationique est choisi dans le groupe constitué par le poly(hexaméthylène biguanine), qui est présent de 0,2 ppm à 2 ppm, le chlorure d'α-[chlorure de 4-tris(2-hydroxyéthyl)ammonium-2-butényl]poly[chlorure de 1-diméthylammonium-2-butényl]-ω-tris(2-hydroxyéthyl)ammonium, qui est présent de 2 ppm à 15 ppm, et l'un quelconque de leurs mélanges.

10. Composition selon les revendications 7 à 9, dans laquelle le fructane modifié par carboxyle ou son sel est une carboxyalkylinuline ou un de ses sels.

11. Composition selon l'une quelconque des revendications 7 à 10, comprenant en outre un ou plusieurs biopolymères et l'un quelconque de leurs dérivés choisis dans le groupe constitué par l'acide hyaluronique et l'alginate.

12. Composition selon l'une quelconque des revendications 7 à 11, comprenant en outre du dexpanthénol, du sorbitol ou l'un quelconque de leurs mélanges.

13. Composition selon l'une quelconque des revendications 7 à 12, comprenant en outre de l'hydroxypropylméthylcellulose, du propylène glycol ou de l'hydroxypropylguar.

14. Composition selon l'une quelconque des revendications 7 à 13, dans laquelle la composition comprend en outre une bétaïne ou un agent tensioactif de sulfobétaïne de formule I 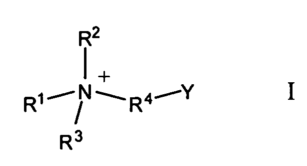 dans laquelle R¹ est R ou -(CH₂)ₙ-NHC(O)R, où R est un groupe alkyle en C₈ à C₃₀ facultativement substitué par un groupe hydroxyle et n vaut 2, 3 ou 4 ; R² et R³ sont chacun choisis indépendamment dans le groupe constitué par un atome d'hydrogène et un groupe alkyle en C₁ à C₄ ; R⁴ est un groupe alkylène en C₂ à C₈ facultativement substitué par un groupe hydroxyle ; et Y est CO₂⁻ ou SO₃⁻.

15. Composition selon la revendication 14, dans laquelle R¹ est R ; R² et R³ sont chacun choisis indépendamment parmi un groupe alkyle en C₁ à C₂; R⁴ est un groupe alkylène en C₂ à C₄ et Y est SO₃⁻.
